# EUROPEAN PATENT APPLICATION

(11) **EP 2 322 912 A1**
(43) Date of publication of application: **18.05.2011**
(21) Application number: 09813081.8
(22) Date of filing: 09.09.2009
(51) Int. Cl.: G01N 21/17, A61B 1/00

(54) **ROTATING LIGHT FIBER UNIT AND OPTICAL COHERENCE TOMOGRAM GENERATING DEVICE**

(30) Priority: 12.09.2008 JP 2008234482; 12.09.2008 JP 2008234483
(71) Applicant: Konica Minolta Opto, Inc., Hachioji-shi, Tokyo 192-8505 (JP)
(72) Inventor: KONNO Kenji, Hachioji-shi Tokyo 192-8505 (JP); JONO Junichi, Hachioji-shi Tokyo 192-8505 (JP)
(74) Representative: Henkel, Feiler & Hänzel
(86) International application number: PCT/JP2009/065719
(87) International publication number: WO 2010/029935

(57) **Abstract**

Provided is a rotary optical fiber unit which can achieve a high coupling efficiency at a low cost and can easily be recovered even when a coupling unit has failed. Provided is also an optical coherence tomographic image forming apparatus using the rotary optical fiber units. A first optical fiber FB1 to transmit a low-coherence light to a second optical fiber FB2 in an optical rotary joint 6 and a cladding of a second optical fiber FB2 are inserted into a capillary 151 so as to couple to each other. The second optical fiber FB2 and a third optical fiber FB3 are detachably attached via a connector unit

## Description

### TECHNICAL FIELD

The present invention relates to a rotation optical fiber unit to radiate light onto a test body while rotating, and an optical coherence tomographic image forming apparatus to radiate a low-coherence light onto the test body via the rotary optical fiber unit and to form a tomography image of the test body from information of light scatted by the test body.

### PRIOR ART

In recent years, to diagnose a physiological tissue, besides an image forming apparatus to obtain optical information of a surface condition of the tissue, an optical coherence tomographic image forming apparatus is suggested. The optical coherence tomographic image forming apparatus is a technology to image a measured portion of the test body wherein, by separating the low-coherence light into two, one separated light is radiated onto the test body the using a rotary optical fiber unit, and by interfering the scattering light to which phase information is added with the other light, phase information of the test body is obtained from intensity information of interfering light (For example, Patent Document 1: Unexamined Japanese Patent Application Publication No. H6-511312). Further to widen the measuring portion, suggested is a technology to rotate an optical probe in the rotary optical fiber unit to propagate the low-coherence light centering around an optical axis. (For example Patent Document 2: Unexamined Japanese Patent Application Publication No. 2007-206049).

### PRIOR ART DOCUMENT

### PATENT DOCUMENT

Patent Document 1: Unexamined Japanese Patent Application Publication No. H6-511312
Patent Document 2: Unexamined Japanese Patent Application Publication No. 2007-206049

### DISCLOSURE OF THE INVENTION

### PROBLEM TO BE SOLVED BY THE INVENTION

To rotate the optical fiber in the optical probe to propagate the low-coherence light, the optical fiber to propagate the light from a light source to the optical probe and an optical fiber in the optical probe has to be coupled with a small eccentricity in order to enhance the coupling efficiency of the optical fibers. Therefore, a rotator to transmit rotation motion of a motor to the optical fiber in the optical probe and the optical fiber in the optical probe have to be assembled with a high accuracy so as to minimize a play, which is very costly.

Also, if a failure occurs at a coupling section between the optical fiber to propagate the light from the light source to the optical probe and the optical fiber in the optical probe, in many cases the failure cannot be recovered only by replacing the optical probe and repairing in a large scale was necessary.

Therefore, an object of the present invention is to provided a rotary optical fiber unit can be easily recovered even in case a failure occurs at the coupling section while achieving a low cost and a high coupling efficiency, and another object of the present invention is to provide an optical coherence tomographic image forming apparatus using the aforesaid rotary fiber unit

### MEANS TO SOLVE THE PROBLEM

Item 1. A rotary optical fiber unit, having: a first optical fiber; a second optical fiber, connected to the first optical fiber; having almost the same cladding diameter as that of the first optical fiber; an optical fiber coupling device provided with an insertion diameter substantially equal to the cladding diameter, having a fine pore to insert each cladding of the first optical fiber and the second optical fiber; an optical probe having; a third optical fiber connected with the second optical fiber, and an optical system fixed at an emitting end of the third optical fiber to radiate an emitted light onto a test body; and a rotation device to rotate the second optical fiber and the third optical fiber.
Item 2. The rotary optical fiber unit of item 1, wherein the optical fiber unit coupling device is fixed at the second optical fiber and configured to be rotatable along with the second optical fiber.
Item 3. The rotary optical fiber unit of item 1 or item 2, wherein the optical probe is configured to be detachable from the second optical fiber.
Item 4. The rotary optical fiber unit of any one of items 1 to 3, further comprising a screw tightening method connector section to couple the second optical fiber with the third optical fiber, wherein when coupling the connector section, a rotation direction to tighten the connector section coincides with a rotation direction in which the second optical fiber and the third optical fiber rotate.
Item 5. The rotary optical fiber unit of any one of items 1 to 4, wherein the first optical fiber is provided with at least one connector section.
Item 6. An optical coherence tomographic image forming apparatus, having: a low-coherence light source, and the rotary optical fiber unit of any one of items 1 to 5 to enter the light from the low-coherence light source,
wherein a tomographic image is formed based on information of light scattered by a test substance.

### EFFECT OF THE INVENTION

Provided are a rotary optical fiber unit can be recovered easily even in case a failure occurs at the coupling section and can achieve a high coupling efficiency at a low cost, and an optical coherence tomographic image forming apparatus using the aforesaid rotary fiber unit

### BRIEF DESCRIPTION OF THE DRAWINGS

Fig. 1a and Fig.1b are diagrams showing a configuration of an optical coherence tomographic image forming apparatus 1 of an embodiment.
Fig. 2 is a diagram showing an outline of an optical rotary joint 6 related to a first embodiment of an optical coherence tomographic image forming apparatus 1.
Fig. 3 is a view showing a coupling section between optical fibers of the embodiment.
Fig. 4 is a diagram showing a framework of the optical probe 8.
Fig. 5 is a view showing an inner scope 31 in which an embodiment is inserted.
Fig. 6 is a view showing an outline of an optical rotary joint 6 related to a second embodiment of an optical coherence tomographic image forming apparatus 1.

### DESCRIPTION OF THE PREFERRED EMBODIMENTS

Embodiments related to the present invention will be described with reference to the drawings without the present invention being limited to the embodiments thereof First, a first embodiment of an optical coherence tomographic image forming apparatus 1 will be described.

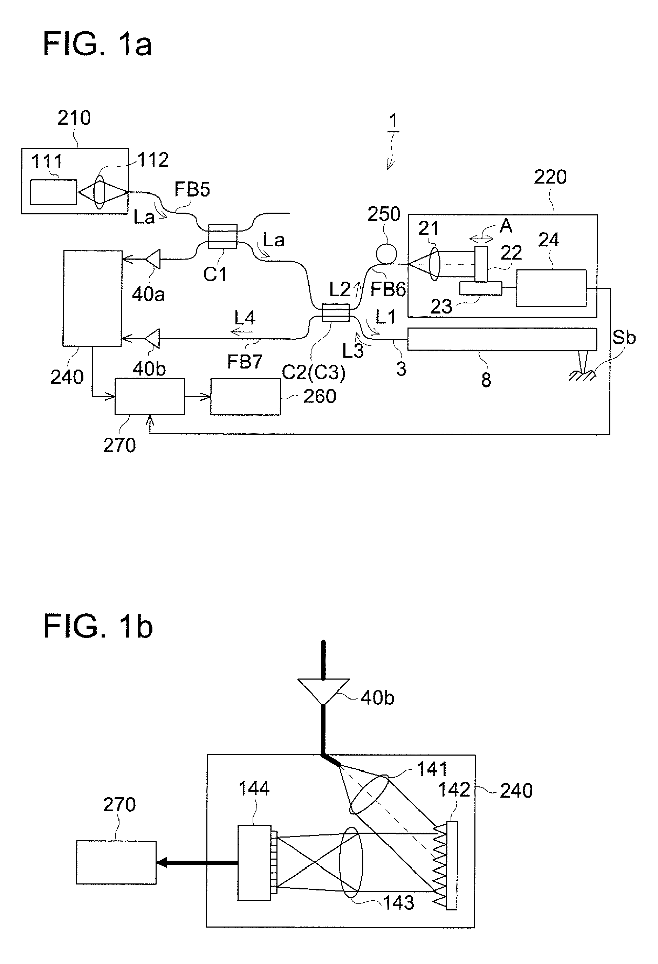

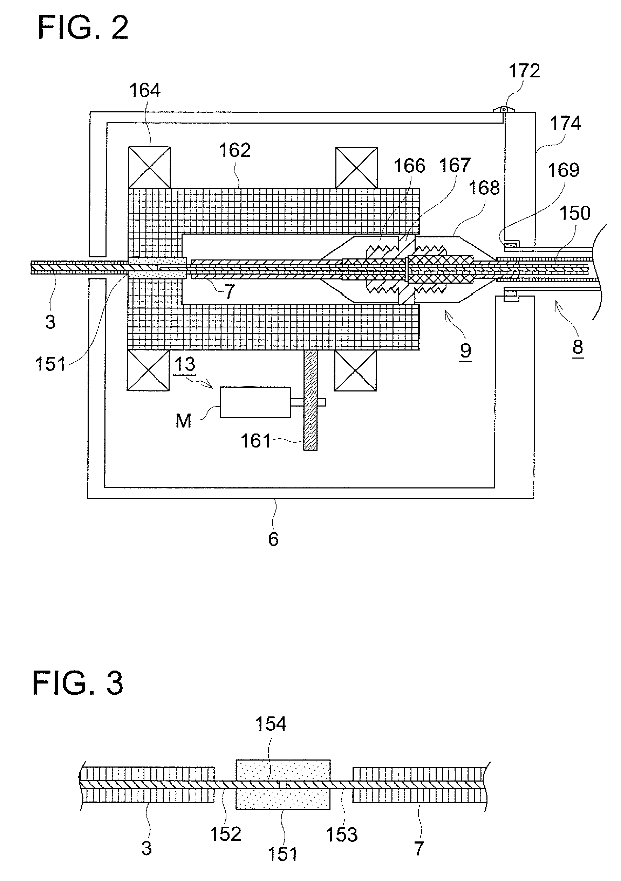

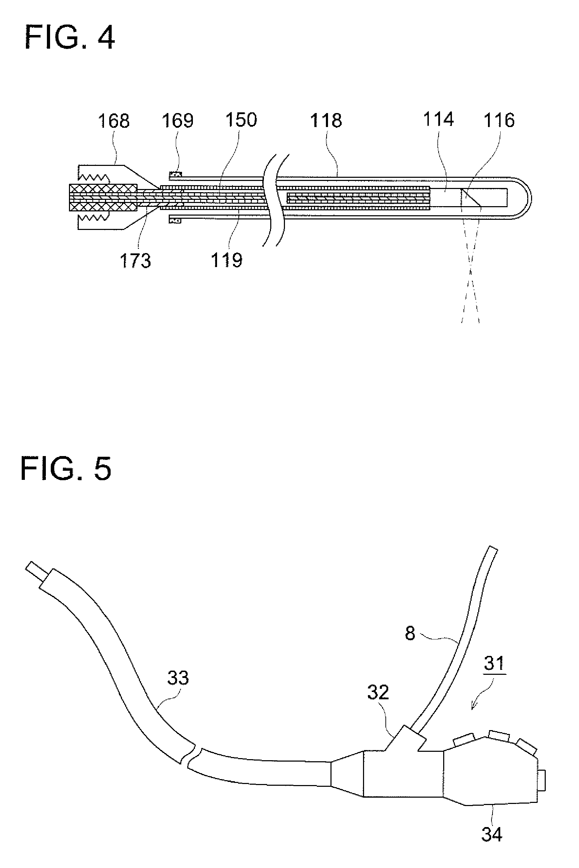

Fig. 1 to Fig. 5 are diagrams to describe the first embodiment. Fig. 1 shows a structure of the optical coherence tomographic image forming apparatus 1 representing the present embodiment, Fig. 2 shows an optical rotary joint 6 to couple the optical fibers rotatably each other which configure the present invention, Fig. 3 shows a coupling section between the optical fibers of the embodiment, Fig. 4 shows a framework of an optical probe 8 and Fig. 5 is a view showing an inner scope 31 in which the present embodiment is to be inserted.

Hereinafter, the optical fiber is a single mold optical fiber. The optical coherence tomographic image forming apparatus 1 shown in Fig. 1(a) acquires a tomographic image of the test body via so called TD (Time Domain) method. The optical coherence tomographic image forming apparatus 1 is provided with a light source unit 210 configured with a light source 111 to emit a low-coherence light LO and an optical system 112,
a light fractionation device C1 to fractionated the low-coherence light LO emitted from the light source unit 210 and propagated in the a light source side optical fiber FL, a light fractionation device C2 to fractionate the low-coherence light LO passed through the optical fractionation device C 1 into a measuring light L1 to irradiate a measuring section and a reference light L2, an optical path adjusting device 220 to adjust an optical path of the reference light L2 which has been fractionated by the light dividing device C2 and propagated in the reference side optical fiber FR, a rotary optical fiber unit 3 to irradiate the test body Sb with the measuring light L1 obtained by fractionating via the light fractionation device C2 while rotating, a multiplexer C3 (light fractionation device C2 doubles) to multiplex an reflected light L3 from the test body and a reflected light L4 from the optical path length adjusting device 220, and
an interfering light detection device 240 to detect an interfering light L5 which is created by multiplexing the reflected light L3 and the reflected light L4 via the multiplexer C3.

The light source unit 210 is provided with a light source 111, such as SLD (Super Luminescent Diode), ASE (Amplified Spontaneous Emission) and a supercontinuum which obtains a broad band light by radiating ultrashort pulse laser light onto a nonlinear medium broadband, and an optical system 112 to conducted the light emitted form the light source into the light source side optical fiber FL.

The above light sources are called a low-coherence light source because of broad wavelength of light source. In case of SLD, the wavelength thereof is 1300m, and SLD is provided with a characteristics of the low-coherence light (hereinafter also called measuring light) that exhibits coherence only in a short distance range such as a coherence length of 17 µm. Namely, in case the light is fractionated into two and thereafter multiplexed again, if a length difference between the two light paths from the dividing point to the multiplexing point is within a short distance range such as around 17 µm, the multiplexed light is detected as an interfered light and if the difference is larger than that, the multiplexed light shows a characteristic of non-interfered light.

The fractionating devices C1 and C2, configured with, for example, optical fibers of 2 x 2, fractionate the low-coherence light LO conducted from the light source 210 via the light source side fiber FL into a measuring light L1 and a reference light L2. The light fractionation device C2 are optically coupled with the rotation optical fiber units 3 and the reference side optical fiber FR respectively and the measuring light L1 propagates in the rotation optical fiber unit 3 and the reference light L2 propagates in the reference side optical fiber FR. Incidentally, the light fractionation device C2 of the present example serves also as the multiplexer C3.

The rotation fiber unit 3 is configured with a first optical fiber FB1, an optical rotary joint 6 and an optical probe 8 (to be described later specifically).

An optical path length adjusting device 220 is configured with a collimator lens 21 to parallelize the reference light L2 emitted form the reference side light fiber FR, a mirror 22 mounded on a base mount movable in an arrow A direction in figure to change the distance from the collimator lens 21, and a mirror moving device 24 to move the base mount 23 and in order to change the measuring position in the measuring subject Sb in the measuring direction, a function to change the length of the light path of the reference light L2 is provided. Further by disposing a phase modulator 250 in the light path (reference side optical fiber FR) of the reference light L2, a function to give a minimal frequency shift with respect to the reference light L2. The reflection light L4 of which light path length has been changed and the frequency has been shifted by the light path length adjusting device 220 is conducted to the multiplexer C3. Through the multiplexer C3, the interfering light L5 created by interfering the reflection light L3 and the reflection light L4 is propagated in the interfering side optical fiber FI, and enters into the detection device 40b to detect the light intensity of the interfering light L5 then subject to photoelectric conversion, and enters into interfering light detection device 240 via.

The interfering light detection device 240 detects light intensity of the interfering light L5 from the multiplexer C3 propagated in the interfering side optical fiber FI via, for example, heterodyne detection. Specifically, in case a total of an entire optical path length of the measuring light L1 and an entire optical path length of the interfering light L3 is equal to entire optical path length of the reference light L2 and the reflected light L4, a bead signal which is alternately weaker and stronger caused by a frequency difference between the reflected lights L4 and L3 is generated. As the optical path length is changed by the optical length adjusting device 220, the measuring position (depth) of the measuring subject is changed, and a plurality of the bead signals at each of positions is detected by the interfering light detection device 240.

The interfering light detection device 240 is connected with an image acquiring device 270 configured with, for example, a computer system such as personal computer, and the image acquiring device 270 is connected with a display device 260 configured with a CRT or a liquid crystal display device.

Meanwhile, information of the measuring position is outputted from the optical path length adjusting device 220 to the image acquisition device 270. Also, in the apparatus of the present embodiment, the detection device 40a to detect the light intensity of the low-coherence light LO fractionated from the light fractionation device C1 of the light source side optical fiber FL and detection device 40b to detect the light intensity of the interfering light L5 are disposed so that the interfering light detection device 240 is provided with a function to adjust a balance of the light intensity of the interfering light L5 based on an output of from the detection device 40a.

Then, based on the bead signal detected via the interfering light detection device 240 and the information of the measuring position in the mirror moving device 24, the image acquisition device 270 creates an optical tomographic image. The created optical tomographic image is displayed on the display device 260.

Next, operation of the optical coherence tomographic image forming apparatus 1 having the aforesaid configuration will be described. When the tomographic image is acquired, first by moving the mirror 22 in the arrow A direction, adjustment of the optical length is carried out so that the measuring substance Sb locates in the measurable range. After that, the light LO is emitted from the light source unit 210, then the light LO is fractionated into the measuring light L1 and the reference light L2 through a light fractionation device C2. The measuring light L1 is emitted towards a body cavity inside and radiated onto the measuring subject Sb. When this occurs, the measuring subject Sb is scanned by the measuring light L1 emitted from an optical probe 8. Then the reflected light L3 from the measuring subject Sb and the reflected light L4 reflected by the mirror 22 are multiplexed and the interfering light L5 of the reflection light L3 and the reflected light L4 is detected by the interference light detection device 240. The detected interfering light L5 is subject to an appropriate wave shape compensation and noise reduction then by Fourier transformation, reflection light intensity distribution information in the measuring direction (depth) of the measuring subject Sb can be obtained.

Then by operating the optical probe 8 to scan the measuring object Sb with the measuring light L1, information of the measuring subject in the measuring direction at each portion along the scanning direction is obtained, thus the tomographic image with respect to a tomographic surface including the aforesaid scanning direction can be obtained. The tomographic image obtained in the above manner is displayed on the display device 260. Incidentally, by moving the optical probe 8 in a left and right direction in Fig. 1a, the measuring subject Sb is scanned by the measuring light L1 in a second direction perpendicular to the aforesaid scanning direction, thus a tomographic image with respect to a tomographic surface including the above second direction can be obtained.

Incidentally, as a method of the optical coherence tomographic c image forming apparatus 1, while so-called a TD method has been quoted to be described, besides the TD method, either so-called SD (Spectral Domain) method to use a spectroscopic system instead of a single detector, or so-called a SS (Swept Source) method to use a wave sweeping laser can be used.

For example, in case of the SD method, the interfering light detection device 240 is to detect the interfering light L5 of reflected light L3 and the reflected light L4 multiplexed by the multiplexer C3, and is provided with a collimator lens 141 to parallelize the interfering light L5 emitted from the interfering side optical fiber F1, a spectroscopic device 142 to disperse the interfering light L5 having a plurality of wavelength bands into each wavelength band, and an light detection device 144 to detect the interfering light L5 having each wave length band dispersed by the spectroscopic device 142 as Fig. 1b shows.

The spectroscopic device 142 configured with, for example, a diffraction grating element and so forth, disperses the interfering light 5L entered therein and emits towards the light detection device 144 via a lens 143. The light detection device 144 is configured with element such as CCD in which optical sensors are arranged, for example, in one or two dimensionally so that each optical sensor detects the interfering light L5 dispersed in the above manner for each the wavelength bands.

Next, the rotary optical fiber unit 3 in the present embodiment will be described. Fig. 2 is an example of an optical rotary joint 6 to couple the optical fibers each other and to enable one side of the optical fiber to rotate. As Fig. 1 shows, a first optical fiber FB 1 is connected with the optical rotary joint 6. In side the optical rotary joint 6, as Fig. 3 shows, trunking of the first optical fiber and the second optical fiber are removed, and a cladding 152 of the first optical fiber FB1 and the cladding 153 of the second optical fiber FB2 are inserted into a fine pore 154 representing a thin pore in a cylindrical shape provided in a capillary 151 and connected directly each other. Incidentally, the capillary 151 having an inserting diameter coincided with a cladding diameter is a collective term of the optical fiber coupling devices to couple and communicated two optical fibers. Thus, any member having an inner diameter in the cylindrical shape, capable of inserting the cladding of the optical fiber therein can be used besides the capillary.

Inside the fine pore 154 of the capillary 151, a core of the first optical fiber FB1 and a core of the second optical fiber FB2 are coupled directly substantially at a center in a longitudinal direction in the fine pore 154 (also called pat coupling). At the contact end surfaces of the cores, the first optical fiber FB 1 and the second optical fiber FB2 are contacted each other perfectly with almost no displacing in axes direction. Also, by filling an unillustrated matching oil, since fresnel reflection at the coupling portion between the first optical fiber FB1 and the second optical fiber FB2 are inhibited, attenuation of the propagation light is suppressed to low levels. Also, change of the optical coupling efficiency of the first optical fiber FB1 and the second optical fiber FB2 due to displacement in the axis direction is very few.

Dimensions of each of sections of the capillary 151 are, for example, an outer diameter of 1 mm, and a length of 50mm. The inner diameter of the capillary 151 is determined to be 126 µm, since the diameters of the cladding of the first optical fiber FB1 and the second optical fiber FB2 are 125 µm to 126 µm including tolerances. In case the optical fibers are connected each other inside the capillary, since the inner diameter of the capillary is larger than the cladding diameter by 1 µm or so, the optical fiber displaces about 1 µm in axis in the capillary 151. However, since the diameter of the cores of the first optical fiber and the second optical fiber is around 10 µm, the eccentricity error falls within 10% of the diameter of the core, thus an excellent coupling efficiency can be obtained.

Contrarily, in case of connecting in a ferrule, since the axial displacement of several millimeters between the ferrules is equal to dire axial displacement between the first optical fiber FB1 and the second optical fiber FB2, occurrence of the same 1 % eccentricity error creates a large loss of the coupling efficiency when coupling directly, because the diameter of the ferrule is large.

Incidentally, characteristics required for the material of the capillary are that the optical fiber is easily laced through the fine pore, a high circularity of the fine pore and a concentricity of the fine pore with respect to the outer circumferential surface are realized accurately by accurate work, a toughness more than some extent is provided and a splinterless against impact is provided. A ceramic sintered material is preferred as the material further provided with a coefficient of thermal expansion close to that of the optical fiber so that the optical fiber does not protrude or retract in the fine pore of the capillary, besides the above characteristics. Specifically, a glass material is preferred from a point of view that the optical fiber can be easily laced through the fine pore. A metal capillary is inferior in workability compared to the ceramic capillary, and high accuracy cannot be realized.

Next, the capillary 151 is fitted with a rotor 162, and further the rotor 162 is supported by bearing 164 and configured to be rotatable. By coupling the second optical fiber FB2 and a third optical fiber FB3 disposed in the optical probe 8 via the connector section 9, the light of the low-coherence light source 2 is transmitted from the first optical fiber FB1 to the second optical fiber FB2 and further transmitted to the third optical fiber FB3.

The connecter section 9 is configured with a FC connecter 166 disposed at the second optical fiber FB2, an adapter 167 and a FC connector 168 disposed at the third optical fiber FB3.

The adapter 167 is detachably fixed at the rotor 162, and the adapter 167 is configured to rotate as the rotor 162 rotates.

Therefore, when the rotor 162 rotates, the capillary 151 and the adaptor 167 rotate and the second optical fiber FB2 also rotates simultaneously. The rotor 162 is driven and rotated by a rotation drive device 13. Specifically, a motor M rotates a roller 161 and the rotor 162 in contact with the roller 161 rotates.

The third optical fiber FB3 is configured to be rotatable in the optical probe 8. Since the third optical fiber FB3 is fixed to the adapter 167 via the FC connecter 168, by rotating the rotor 162, the third optical fiber FB3 rotates. The second optical fiber FB2 and the third optical fiber FB3 are coaxial with each axis center and rotate centering around the axis center as a rotation axis.

By dismounting the FC connector 168 from the adaptor 167, the third optical fiber FB3 and the second optical fiber FB2 become detachable. Also, since a stop ring 169 is provided at the optical probe 8, so that the optical probe 8 can be detached with an outer section of the optical rotary joint 6, by detaching the FC connector 168 and the stop ring 169, the optical probe 8 can be detached from the second optical fiber FB2 and the optical rotary joint 6. Thus, when the optical probe 8 fails, it can be replaced.

Also, by detaching the adapter 167 from the rotor 162, in case a damage occurs at the cladding of the second optical fiber FB2 in the capillary 151, the second optical fiber FB2 can be replaced readily.

Utilizing a screw tightening coupling method such as the above configuration for the connection section 9 is preferable. The screw tightening coupling method is a method wherein a male screw is formed on one side of the connector to be coupled and a female screw is formed on another side, then the couplers are coupled by tighten the screws. In the screw tightening coupling method, since the screws are tightened by rotation, if the connector is rotated in a reverse direction to tighten the screw the, the screws may loose. Thus the shapes of the screws are formed so that the tightening rotation direction of the screws coincides with the rotation direction of the third optical fiber FB3.

Next, Fig. 4 shows a structural view of the optical prove 8 from the connector section 9 to a front end. The light entered into the third optical fiber FB3 from the second optical fiber FB2 enters into a GNIN lens 114 disposed at a front end of the third optical fiber FB3, and is converged via the GRIN lens 114. After being emitted from the GRIN lens 114, the light path of the light is bended 90 via a right angle prism 116.

A sheath 118 is a flexible tube retaining the third optical fiber FB3, the GRIN lens 114 and the right angle prism 116 inside configured with a material having high transparence such as Tefron^{™} through which the low-coherence light particularly can transmit with a high efficiency. The measuring light L1 transmits the sheath 118 and via a function of the right angle prism 116, the light L1 is emitted from a side surface thereof to an outside. Incidentally, silicone family oil is filled in the sheath 118 so that a difference of refraction index between the right angle prism 116 and a space in the sheath 118 is reduced.

A torque wire is 119 is provided inside the sheath. The torque wire 119 is a steel wire winded around the cladding to transmit a torque transmitted from the rotor 162 to the connector 9 to the right angle prism 116 and the GRIN lens 114 and to prevent the cladding from breakage.

The measuring light L1 converges inside the body tissue via power of the GRIN lens 114 via the right angle prism 116. Then the measuring light L1 is reflected by the body tissue inside and enters in the right angle prism 116 via the sheath 118, then the light path thereof bends 90°. Then the measuring light L1 enters into the GRIN lens 114, the third optical fiber FB3, the second optical fiber FB2 and the first optical fiber FB1.

The third optical fiber FB3 of the optical probe 8 and the right angle prism 116 and the GRIN lens 114 fixed onto the third optical fiber FB3 radiates the measuring light L1 onto the body tissue inside while rotating around the axis of the third optical fiber FB3, and the measuring light L1 is emitted towards the body tissue located along a radial direction of the optical probe to perform scanning (namely radial scanning).

The rotation drive device 13 to rotate the third optical fiber FB3, the right angle prism 116 and the GRIN lens 114 is connected with an unillustrated control section 25 electrically For example, when an unillustrated switch for the radial scanning disposed at the control section 25 is turned on, the rotation drive device 13 outputs a drive pulse to control driving under the control of the control section 25.

A lid section 174 configured to open and close via a hinge 172 is disposed at an outer covering section of the optical rotary joint 6. The optical probe 8 is engaged by a stop ring on the outer covering section of the optical rotary joint 6. A user can opens the lid 174 and remove the optical prove 8 from the connection section 9.

Next an inner scope 31 attached to the optical coherence tomographic image forming apparatus 1 will be described. As Fig. 5 shows, in the optical probe 8 in the embodiment can be inserted into a forceps insertion opening 32 of the inner scope 31, and through a channel to insert the forceps, the front end side of the optical probe 8 can be protruded from a front end opening of the channel thereof

The inner scope 31 has an elongated insertion section 33 so that the inner scope can readily inserted into the body cavity, and at a rear end of the insertion section 33 an operation section 34 having a thick width is provided. At a vicinity of the rear end of the insertion section, a forceps insertion opening 32 is provided, the forceps insertion opening 32 is communicated with the forceps insertion channel therein.

In the insertion section 33, an unillustrated light guide is inserted By connecting an incident light end of the light guide with a light source device, a transmitted illuminating light is emitted from an illuminating light window provided at an end of the insertion section 33 to illuminate an affected area. Also, an observation window is disposed adjacent to the illuminating light window. At the observation window an objective optical system is provided so that the affective area can be observed through the optical system,

Under the observation via the observation optical system at the front end of the inner scope 31, the low-coherence light is radiated from the optical prove 8 onto the focused portion of the effected area of the body tissue 11 side, then a tomographic image data of the measuring subject Sb inside is acquired, and then an optical tomographic image is displayed on a display surface of the monitor 26.

As above, according to the first embodiment, the first optical fiber FB1 and the second optical fiber FB2 having the almost same cladding diameter as that of the first optical fiber FB1 are inserted and connected in the capillary 151, whereby the rotary optical fiber units 3 whi ch achieved a low cost and a high coupling efficiency can be provided and the optical coherence tomographic image forming apparatus 1 using the aforesaid rotary optical fiber unit 3 can be provided.

Meanwhile, the second optical fiber FB2 can be fixed onto the capillary 151 by bonding. By bonding the second optical fiber FB2 on the capillary 151, twisting of the second optical fiber due to rotation of the FC connecter 166 can be inhibited and a distance between the second optical fiber FB2 and the first optical fiber FB1 can be maintained constant, thus change of coupling efficiency can be inhibited.

Also, the rotary optical fiber 3 of the present invention can be used in a scanning system of fluorescence measurement and spectrometric measurement. In the fluorescence measurement wherein by radiating the exciting light, a fluorescence generated by the test body is acquired, by inserting the optical probe inside the test body and rotating the optical probe, a periphery of the inserted probe can be measured.

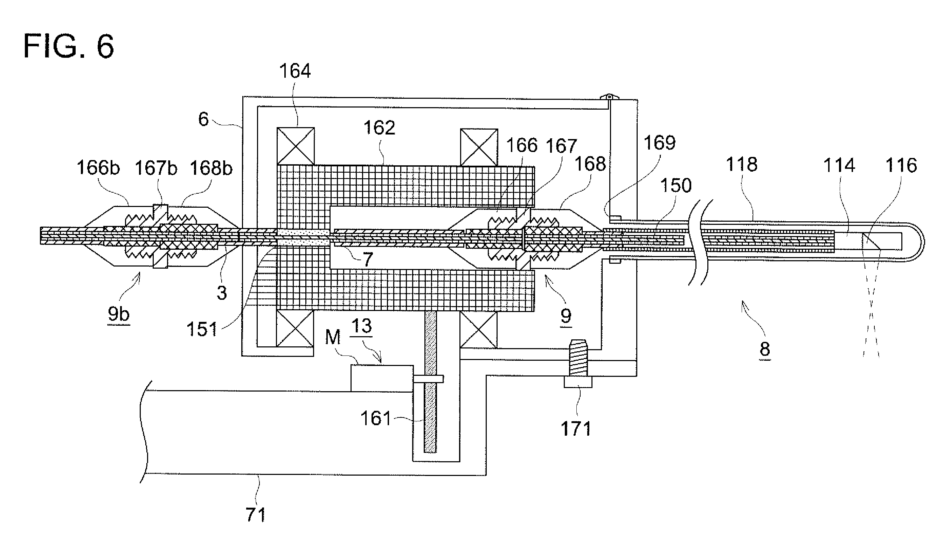

Next, a second embodiment of the optical coherence tomographic image forming apparatus will be described with reference to Fig. 6. Fig. 6 shows a coupling portion of the optical rotary joint 6. In the second embodiment, the optical probe 8 is configured to be replaceable. As Fig. 6 shows, a coupling section is provided by the connecter section 9b to somewhere between the optical rotary joint 6 and the first optical fiber FB1 on the low-coherence light source 2 side. By providing the coupling section a front end section can be replaced from the connection section 9b. A plurality of the connecter sections 9b can be provided at a plurality of the positions. The connecter section 9b is configured with a FC connecter 168b provided on the optical rotary joint 6 side, a FC connector 166b provided on the optical coherence tomographic image forming apparatus main body 71 side, and an adaptor 167b.
The FC connector 168b can be removed from the adapter 167b by rotating the FC connector 168b. The optical rotary joint 6 is, for example, configured to be fixed onto the optical coherence tomographic image forming apparatus main body 71 by a screw 171, and the by removing the screw 171, the optical rotary joint 6 is detached form the optical coherence tomographic image forming apparatus main body 71. The rotation drive device 13 is configured to be provided on the optical coherence tomographic image forming apparatus main body 71 side.

As above, according to the second embodiment, by providing the connector section 9b on the optical coherence tomographic image forming apparatus main body 71 side from the optical rotary joint 6, even in case that inner part of the optical rotary joint 6 is needed to be replaced due to a failure in the capillary 151 caused by increase of usage of the optical probe 8, by replacing everything from the connector section 9b to the optical probe 8, which saves labor to repair the optical rotary joint 6, the rotary optical fiber unit 3 capable of addressing the failure early at a low cost can be provided and the optical coherence tomographic image forming apparatus using the rotary fiber units 3 can be provided.

### DESCRIPTION OF THE SYMBOLS

1 Optical coherence tomographic image forming apparatus
2 Low-coherence light source
3 Rotary fiber unit
6 Optical rotary joint
8 Optical probe
9 Connector section
10 Optical probe
11 Body tissue
13 Rotation drive device
21 Collimator lens
22 Mirror
23 Base mount
24 Mirror moving device
25 Control section
26 Monitor
31 Inner scope
32 Forceps insertion opening
33 Insertion section
34 Operation section
40 Detection device
71 Optical coherence tomographic image forming apparatus
91 Connector section
111 Light source
112 Optical system
114 GRIN lens
116 Right angle prism
118 Sheath
119 Torque wire
142 Spectral device
144 Light detection device
151 Capillary
152 Cladding
153 Cladding
154 Fine pore
161 Roller
162 Rotor
166 FC connector
167 Adaptor
168 FC connector
169 Ring
171 Screw
172 Hinge
174 Lid section
210 Light source unit
220 Light path length adjusting device
250 Phase modulator
270 Image acquisition device
C1 Light fractionation device
C2 Light fractionation device
C3 Multiplexer
FL Light source side optical fiber
FR Reference side optical fiber
FI Interfering side optical fiber
FB1 First optical fiber
FB2 Second optical fiber
FB3 Third optical fiber

## Claims

1. A rotary optical fiber unit, comprising:
a first optical fiber,
a second optical fiber, connected to the first optical fiber, having almost the same cladding diameter as that of the first optical fiber,
an optical fiber coupling device provided with an insertion diameter substantially equal to the cladding diameter, having a fine pore to insert each cladding of the first optical fiber and the second optical fiber,
an optical probe having;
a third optical fiber connected with the second optical fiber, and
an optical system fixed at an emitting end of the third optical fiber to radiate an emitted light onto a test body, and
a rotation device to rotate the second optical fiber and the third optical fiber.

2. The rotary optical fiber unit of claim 1, wherein the optical fiber unit coupling device is fixed at the second optical fiber and configured to be rotatable along with the second optical fiber.

3. The rotary optical fiber unit of claim 1 or claim 2, wherein the optical probe is configured to be detachable from the second optical fiber.

4. The rotary optical fiber unit of any one of claims 1 to 3, further comprising a screw tightening method connector section to couple the second optical fiber with the third optical fiber, wherein when coupling the connector section, a rotation direction to tighten the connector section coincides with a rotation direction in which the second optical fiber and the third optical fiber rotate.

5. The rotary optical fiber unit of any one of claims 1 to 4, wherein the first optical fiber is provided with at least one connector section.

6. An optical coherence tomographic image forming apparatus, comprising:
a low-coherence light source, and
the rotary optical fiber unit of any one of claims 1 to 5 to enter the light from the low-coherence light source,
wherein a tomographic image is formed based on information of light scattered by a test substance.
